(19) Europäisches Patentamt
European Patent Office
Office européen des brevets

(11) Veröffentlichungsnummer: **0 087 659**
**B1**

# (12) EUROPÄISCHE PATENTSCHRIFT

(45) Veröffentlichungstag der Patentschrift: **01.04.87**

(51) Int. Cl.⁴: **C 07 D 207/26**, C 07 D 209/48, C 07 D 233/32, C 07 D 263/22, C 08 F 26/06, B 01 J 41/00

(21) Anmeldenummer: **83101357.8**

(22) Anmeldetag: **12.02.83**

## (54) Verfahren zur Herstellung von cyclischen N-Vinylacylaminen.

(30) Priorität: **26.02.82 DE 3207031**

(43) Veröffentlichungstag der Anmeldung: **07.09.83 Patentblatt 83/36**

(45) Bekanntmachung des Hinweises auf die Patenterteilung: **01.04.87 Patentblatt 87/14**

(84) Benannte Vertragsstaaten: **DE FR GB IT**

(56) Entgegenhaltungen:
**FR - A - 1 361 270**
**FR - A - 2 103 021**
**GB - A - 807 016**
**US - A - 2 669 570**
**US - A - 2 840 566**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Krimm, Heinrich, Dr., Heyenbaumstrasse 65, D-4150 Krefeld (DE)**
Erfinder: **Buysch, Hans-Josef, Dr., Brandenburger Strasse 28, D-4150 Krefeld (DE)**
Erfinder: **Lange, Peter Michael, Dr., Walter-Flex-Strasse 9, D-5090 Leverkusen (DE)**
Erfinder: **Klipper, Reinhold, Dr., Bismarckstrasse 13, D-5000 Köln 50 (DE)**

**EP 0 087 659 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung cyclischer N-Vinylacylamine.

Aus Ann. 601, 81 (1956) ist bekannt, cyclische N-Vinylacylamine durch Umsetzung cyclischer Arylamine mit Acetylen oder Acetylenderivaten bei höheren Temperaturen vorzugsweise in Druckgefässen herzustellen. Nachteilig bei diesem Verfahren sind die umfangreichen Sicherheitsmassnahmen und die technisch aufwendigen Apparaturen, die für die Handhabung des Acetylens erforderlich sind.

In der GB-A 807 016 und US-A 2 840 566 wird ein Verfahren zur Herstellung von N-Vinyl-2-oxazolidinon bzw. N-Vinylethylenharnstoff beschrieben. Die dort beschriebenen Verfahren sind dadurch gekennzeichnet, dass die entsprechenden N-β-Hydroxyethylverbindungen mit toxischem Thionylchlorid zu den N-β-Chlorethylverbindungen umgesetzt werden. Die N-β-Chlorethylverbindungen werden dann in einem weiteren Verfahrensschritt mit einer Base zur Reaktion gebracht, wobei neben den N-Vinylverbindungen zwangsweise stöchiometrische Mengen an umweltbelastenden Salzen anfallen.

Weiterhin ist aus der US-Patentschrift 2 669 570 bekannt, N-Vinylpyrrolidone herzustellen, in dem die entsprechenden N-β-Hydroxyethylpyrrolidone bei vermindertem Druck in der Gasphase bei 300 bis 340°C über aktiviertes Aluminium geleitet werden. In der FR-A 1 361 270 wird darüber hinaus ein Verfahren zur Herstellung von N-Vinylimiden beschrieben, das dadurch gekennzeichnet ist, dass man entsprechende N-α-Acetoxyethylenverbindungen sekundärer Alkohole einer katalytischen Spaltung (Säurekatalysator) unterwirft. Die Spaltung der dort eingesetzten N-α-Acetoxyethylenverbindungen verläuft nicht so glatt, wie dem Beispiel 4 (Herstellung von N-Vinyl-phthalimid) entnommen werden kann. Die erhaltenen Ausbeuten an N-Vinyl-phthalimid betragen nämlich nur 26%.

Es wurde nun ein Verfahren zur Herstellung cyclischer N-Vinylacylamine der Formel (I)

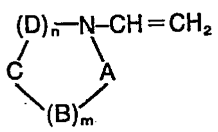
(I),

worin

A und D die CO-Gruppe bedeuten,

B für Sauerstoff oder die NH-Gruppe steht,

C einen gesättigten Kohlenwasserstoffrest mit 1 bis 3 Kohlenstoffatomen oder einen Benzolring, der teilweise oder vollständig hydriert sein kann, darstellt

und

m und n unabhängig voneinander 0 oder 1 bedeuten und wobei die Zahl der Ringglieder 5 oder 6 ist,

gefunden, das dadurch gekennzeichnet ist, dass man Kohlensäureester von cyclischen N-Hydroxyethyl-acylaminen der Formel (II) oder (III)

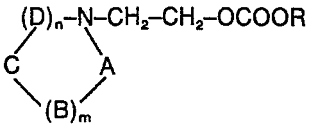
(II)

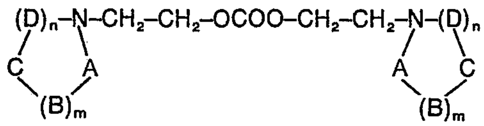
(III),

in denen

A, B, C, D, m und n die zuvor genannte Bedeutung haben und

R für einen gesättigten Kohlenwasserstoffrest mit 1 bis 4 Kohlenstoffatomen steht, in Gegenwart katalytischer Mengen einer oder mehrerer alkalisch reagierender Verbindungen von Alkali- oder Erdalkalimetallen auf Temperaturen von 120 bis 250°C erhitzt und anschliessend das Reaktionsprodukt durch Destillation abtrennt.

Als gesättigte Kohlenwasserstoffreste mit 1 bis 4, bevorzugt 1 bis 2, Kohlenstoffatomen der Formel (II) seien genannt: Der Methyl-, der Ethyl-, der N-Propyl-, der Iso-propyl-, der N-Butyl-, der Isobutyl- und der tert.-Butyl-Rest, bevorzugt der Methyl- und der Ethyl-Rest.

Beispielsweise können folgende Kohlensäureester cyclischer N-2-Hydroxyethyl-acylamine in das erfindungsgemässe Verfahren eingesetzt werden:

Die Carbonate oder die Alkylcarbonate,
wie die Methyl- oder Ethylcarbonate,
des N-2-Hydroxyethyl-pyrrolidinons,
des N-2-Hydroxyethyl-piperidinons,
des N-2-Hydroxyethyl-oxazolidinons,
des N-2-Hydroxyethyl-tetrahydrooxazinons,
des N-2-Hydroxyethylimidazolidinons,
des N-2-Hydroxyethyl-succinimids.
des N-2-Hydroxyethyl-phthalimids,
des N-2-Hydroxyethyl-tetra-hydrophthalimids
und des N-2-Hydroxyethyl-hexahydrophthalimids,
bevorzugt des N-2-Hydroxyethyl-pyrrolidinons,
N-2-Hydroxyethyl-oxazolidinons und des
N-2-Hydroxyethyl-phthalimids.

Als alkalisch reagierende Verbindungen von Alkali- oder Erdalkalimetallen seien genannt: Die Hydroxide, Alkoholate oder die alkalisch reagierenden Salze, wie die Carbonate oder die Carboxylate des Lithiums, Natriums, Kaliums, Magnesiums, Calciums, Strontiums oder des Bariums. Bevorzugt werden eingesetzt die Carbonate, die Carboxylate, die Alkoholate oder die Hydroxide des Natriums oder des Kaliums. Besonders bevorzugt wird Natriumhydroxid oder Kaliumcarbonat eingesetzt.

Bevorzugt werden die alkalisch reagierenden Verbindungen der Alkali- oder Erdalkalimetalle in Mengen von 0,001 bis 5 Gew.-%, besonders bevorzugt in Mengen von 0,001 bis 1 Gew.-%, bezogen auf die Menge des Ausgangsmaterials, eingesetzt.

Das erfindungsgemässe Verfahren wird bei Temperaturen von etwa 120 bis 250°C, vorzugsweise bei 140 bis 220°C, durchgeführt.

Man isoliert die Reaktionsprodukte in bevorzugter Weise durch Destillation unter vermindertem Druck bei 0,01 bis 200, bevorzugt bei 0,01 bis 25 mbar.

Die erfindungsgemäss einzusetzenden Kohlensäureester von cyclischen N-Hydroxyethyl-acylaminen erhält man in bekannter Weise durch z.B. Umsetzung der N-2-Hydroxyethyl-acylamine mit Kohlensäurederivaten, wie Dimethyl- oder Diethylcarbonat, oder durch Umsetzung von Dialkanolaminen oder von Aminoalkyl-aminoalkanolen mit beispielsweise Diethylcarbonat in Gegenwart von Umesterungskatalysatoren wie der alkalisch reagierenden Verbindungen der Alkali- oder Erdalkalimetalle.

Je nachdem, ob als Ausgangsstoffe für das erfindungsgemässe Verfahren Carbonate oder Alkylcarbonate der cyclischen N-2-Hydroxyethylacylamine eingesetzt werden, verläuft die Umsetzung in verschiedener Weise. Carbonate werden bei den angegebenen Reaktionstemperaturen in Gemische von cyclischen N-Vinyl-acylaminen (I) und cyclischen N-2-Hydroxyethyl-acylaminen (IV) unter Decarboxylierung gespalten (s. nachfolgendes Formelschema):

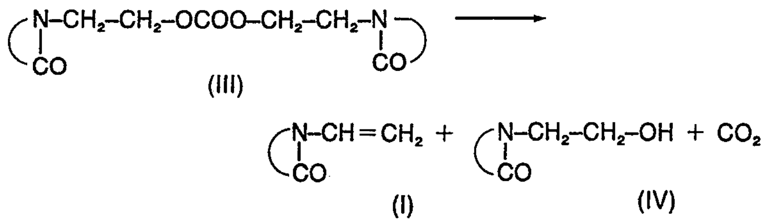
(III) (I) (IV)

Alkylcarbonate liefern stattdessen N-Vinylacylamine und Alkohole (s. nachfolgendes Formelschema):

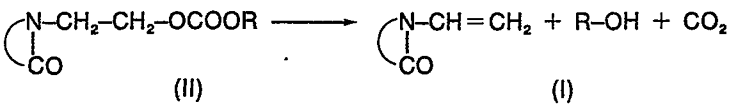
(II) (I)

In den meisten Fällen wird neben dieser Reaktion auch ein Übergang vom Alkylcarbonat des cyclischen N-2-Hydroxyethyl-acylamins in das entsprechende Carbonat unter Abspaltung von Diethylcarbonat beobachtet (s. nachfolgendes Formelschema):

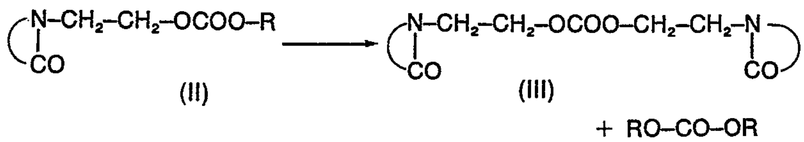
(II) (III)

Daher findet sich im Reaktionsgemisch ein mehr oder weniger grosser Anteil an cyclischen N-2-Hydroxyethyl-acylaminen der Formel (IV).

Der beim erfindungsgemässen Verfahren abgespaltene Alkohol, beispielsweise Methanol oder Ethanol, wird zweckmässigerweise in einer mit Trockeneis beschickten Kühlfalle aufgefangen, während das Kohlendioxid in einer mit flüssigem Stickstoff gekühlten Falle absorbiert wird. Das Kohlendioxid kann auch in einer geeigneten Flüssigkeit, wie einer konzentrierten Alkalilauge oder in einem höher siedenden primären Amin, wie Aminoethanol oder Ethylendiamin, unter Carbaminatbildung aufgefangen werden.

Die durch Destillation nach dem erfindungsgemässen Verfahren erhaltenen Reaktionsprodukte sind, wie zuvor ausgeführt, in der Regel Gemische aus den Verbindungen der Formel (I) und (IV). Diese werden üblicherweise durch fraktioniertes Umkristallisieren und/oder durch Destillation getrennt. Die cyclischen N-2-Hydroxyethyl-acylamine werden in bevorzugter Weise wieder in die Reaktion zurückgeführt.

Die cyclischen N-Vinylacylamine sind wertvolle Vorprodukte zur Herstellung von Pflanzenschutzmitteln und Arzneimitteln und dienen als Polymerisations- und Copolymerisationskomponenten zur Herstellung von Kunststoffen, Überzügen, Lacken und Ionenaustauschern (vgl. z.B. US 2 276 840, DE-PS 832 437, US 2 818 362 und Ullmanns Encycl. Techn. Chem., 4. Aufl. (1972) Bd. 19, 385 (1980).

Die nachfolgenden Beispiele sollen die Herstellung der cyclischen N-Vinylacylamine sowie deren Verwendung zur Herstellung von Ionenaustauschern verdeutlichen.

I. Herstellung von cyclischen N-Vinylacylaminen

Beispiel 1

a) N-2-Oxazolidinonyl-ethyl-ethylcarbonat

210 g (2 Mol) Diethanolamin, 1180 g (10 Mol) Diethylcarbonat und 2 g Kaliumcarbonat erhitzt man unter Rühren an einer Füllkörperkolonne 2 Stunden auf 102 bis 130°C Innentemperatur, während über Kopf 270 g Alkohol abgespalten werden. Man entfernt den Katalysator, indem man das Reaktionssprodukt über eine mit 200 g eines Ionentauschers, eines sulfonierten Polystyrolharzes, beschickte Säule laufen lässt. Nach Abdestillieren des überschüssigen Diethylcarbonats wird das N-2-Oxazolidinonyl-ethyl-ethylcarbonat bei 130 bis 133°C/0,02 mbar destilliert. Ausbeute 375 g = 92% der Theorie; Schmp. 32 bis 33°C.

b) N-Vinyloxazolidinon

60 g des Reaktionsproduktes aus a) werden nach Zugabe von 5 mg Natriumhydroxid unter einem Druck von 0,03 mbar auf 136 bis 160°C Innentemperatur erhitzt, während lebhaft Kohlendioxid abgespalten wird und bei 100 bis 136°C ein farbloses Destillat (16 g) übergeht. In einer mit Trockeneis beschickten Kühlfalle wird ein Gemisch von Ethanol und Diethylcarbonat, in einer Kühlfalle mit flüssigem Stickstoff das Kohlendioxid aufgefangen. Der Destillationsrückstand (25 g) kristallisiert beim Abkühlen. Schmp. 107°C. Er besteht aus Di-(N-2-oxazolidinonyl-ethyl)-carbonat. Durch Redestillation erhält man bei 60 bis 62°C/0,01 mbar 12 g N-Vinyloxazolidinon, $n_D^{20} = 1.4970$; Schmp. −6°C.

Beispiel 2

a) Di-(N-2-oxazolidinonyl-ethyl)-carbonat

1650 g (16 Mol) Diethanolamin, 3115 g (26,4 Mol) Di-ethylcarbonat und 4 g Kaliumcarbonat werden an einer 1,10-m-Füllkörperkolonne während 7 Stunden auf 105 bis 132°C Innentemperatur erhitzt, bis über Kopf 1884 g Ethanol abdestilliert sind. Den Rest des Alkohols (315 g) trennt man durch stufenweises Verringern des Drucks bis auf 80 mbar ab. Nach Abdestillieren des überschüssigen Diethylcarbonats unter 30 mbar erhält man 2370 g Rückstand, der nahezu aus reinem Di-(N-2-oxazolidinonyl-ethyl)-carbonat besteht. Schmp. 107°C (aus Essigester umkristallisiert), Sdp. 230°C/0,01 mbar.

$C_{11}H_{16}N_2O_7$ (288,2)

| | | | |
|---|---|---|---|
| ber. | C 45,83 | H 5,56 | N 9,72 |
| gef. | C 45,7 | H 5,62 | N 9,69 |

b) N-Vinyloxazolidinon

In einen auf 190°C geheizten Rührkolben lässt man im Laufe von 4 Stunden 295 g des nach a) erhaltenen Produktes unter einem Druck von 0,03 mbar eintropfen, während das Reaktionsprodukt bei 140 bis 160°C übergeht. Das Kohlendioxid wird in einer mit flüssigem Stickstoff gekühlten Falle aufgefangen. Die Menge des Destillats beträgt 210 g, die des Kohlendioxids 41 g. Durch Redestillation erhält man 2 Fraktionen, bei 80 bis 85°C/0,01 mbar 102 g N-Vinyloxazolidinon und bei 145 bis 156°C/0,01 mbar 103 g N-2-Hydroxyethyl-oxazolidinon. Ausbeute an N-Vinyloxazolidinon 90% der Theorie; $n_D^{20} = 1.4968$. Das gleichzeitig erhaltene N-2-Hydroxyethyl-oxazolidinon, Schmp. 32 bis 33°C, $n_D^{20} = 1.4830$, kann wieder mit Diethylcarbonat zum Di-(N-2-oxazolidinonyl-ethyl)-carbonat umgesetzt werden.

Beispiel 3

a) Hydroxyethylphthalimid

Man trägt 888 g (6 Mol) Phthalsäureanhydrid portionsweise in 412,5 g 98%iges Ethanolamin (6,6 Mol) ein und erhitzt innerhalb 2 Stunden von 110 auf 190°C, bis 114 g Wasser abdestilliert sind. Man entfernt dann das überschüssige Ethanolamin unter vermindertem Druck bis 145°C/0,1 mbar. Die Ausbeute ist quantitativ: 1145 g.

b) N-2-phthalimidyl-ethyl-ethylcarbonat

In der gleichen Apparatur wie in Beispiel 2a) erhitzt man 1145 g (6 Mol) Hydroxyethylphthalimid, 2832 g (24 Mol) Diethylcarbonat und 3 g Kaliumcarbonat während 3,5 Stunden auf 127 bis 140°C Innentemperatur, bis 276 g Ethanol über Kopf abgetrennt sind. Unter vermindertem Druck (200–30 mbar) destilliert man sodann bis 125°C Innentemperatur 2200 g Diethylcarbonat ab und erhält 1525 g eines kristallisierten Rückstandes, der im wesentlichen aus N-2-Phthalimidylethyl-ethylcarbonat besteht. Schmp. 106°C (aus Toluol umkristallisiert).

c) N-Vinylphthalimid

Das Produkt aus b) erhitzt man in einem Kolben mit weiter Claisen-Brücke auf 160 bis 200°C Innentemperatur, während Alkohol, Diethylcarbonat und Kohlendioxid abgespalten werden und bei 140 bis 160°C/0,07 mbar ein in der Vorlage erstarrendes Destillat übergeht. Das Destillat (950 g) wird in der 3fachen Menge Toluol gelöst. Beim Abkühlen fällt N-2-Hydroxyethyl-phthalimid aus. Das Filtrat wird nach Zusatz von 5 g Kupferpulver redestilliert. Man erhält bei 90 bis 95°C/0,04 mbar 503 g N-Vinylphthalimid; Schmp. 80°C. Eine weitere bei 151 bis 153°C/0,04 mbar übergehende Fraktion besteht aus Hydroxyethylphthalimid. Insgesamt werden 400 g Hydroxyethylphthalimid zurückgewonnen. Ausbeute an N-Vinylphthalimid, bezogen auf umgesetztes Hydroxyethylphthalimid, 75%.

Beispiel 4

a)N-2-Hydroxyethyl-ethylenharnstoff (-imidazolidinon)

Man erhitzt 104 g (1 Mol) 2-(2-Amino-ethylamino)-ethanol und 60 g Harnstoff auf 90 bis 230°C, bis die Abspaltung von Ammoniak beendet ist, und erhält in der berechneten Ausbeute N-2-Hydroxyethyl-imidazolidinon.

b) N-Vinylimidazolidinon

130 g (1 Mol) N-2-Hydroxyethyl-imidazolidinon, 472 g (4 Mol) Diethylcarbonat und 1 g Kaliumcarbonat werden wie in Beispiel 2a) auf 110 bis 130°C erhitzt, am Schluss unter 400 mbar, bis die berechnete Menge Ethanol abgespalten ist. Das Reaktionsprodukt wird filtriert und unter 220 mbar vom überschüssigen Diethylcarbonat befreit. Der Rückstand (125 g) wird nach Zusatz von 2 g Kupferpulver auf 200 bis 210°C/0,04 mbar erhitzt, während Kohlendioxid und Ethanol abgespalten werden und bei 140 bis 150°C/0,04 mbar ein teilweise kristallisiertes Destillat übergeht. Durch Redestillation werden bei 104 bis 116°C/0,04 mbar 12 g N-Vinylimidazolidinon erhalten. Schmp. 77 bis 79°C (aus wenig Methanol umkristallisiert).

Beispiel 5

a) N-2-Hydroxyethyl-pyrrolidinon

Man lässt unter gelindem Kühlen 430 g (5 Mol) Butyrolacton in 403 g (6,5 Mol) Aminoethanol einlaufen und treibt das Reaktionswasser und überschüssiges Aminoethanol durch Erhitzen auf 180 bis 190°C, am Schluss bei 20 mbar, innerhalb 36

Stunden über. Bei 119 bis 123°C/0,02 mbar gehen 550 g (= 85% der Theorie) Hydroxyethylpyrrolidinon, $n_D^{20}$ = 1.4950, über.

b) N-2-Pyrrolidinonyl-ethyl-ethylcarbonat

Man errhitzt 258 g (2 Mol) Hydroxyethyl-pyrrolidinon, 708 g (6 Mol) Diethylcarbonat und 1 g Kaliumcarbonat wie in Beispiel 1a) auf 110 bis 130°C und lässt über Kopf die berechnete Menge Alkohol übergehen. Unter vermindertem Druck wird das nicht-umgesetzte Diethylcarbonat bis 130°C entfernt. Man erhält 380 g N-2-Pyrrolidinonyl-ethyl-ethylcarbonat von Sdp. 98°C/0,02 mbar; $n_D^{20}$ = 1.4656.

c) N-Vinylpyrrolidinon

Das Produkt von b) wird innerhalb von 11 Stunden unter 20 mbar auf 150 bis 160°C erhitzt und bei 90 bis 160°C 220 g Destillat abgetrennt. Durch Redestillation über eine 15 cm-Vigreux-Kolonne erhält man 30 g Diethylcarbonat vom Sdp. 24 bis 27°C/16 mbar, 92 g N-Vinylpyrrolidinon, 92 bis 93°C/16 mbar, und 42 g Hydroxyethylpyrrolidinon, 112 bis 116°C/0,24 mbar. Ausbeute 50%, bezogen auf umgesetztes Hydroxyethylpyrrolidinon.

II. Verwendung von cyclischen Vinylacylaminen zur Herstellung von Ionenaustauschern (am Beispiel des N-Vinylphthalimids gezeigt)

Herstellung der Perlpolymerisate

Beispiel 6

In einem Reaktionsgefäss, bestückt mit Thermometer, Rührer und Rückflusskühler, werden 500 ml destilliertes Wasser und 0,27 g Methylcellulose vorgelegt und auf 60°C erwärmt. Dazu gibt man unter Rühren die gelöste organische Phase, bestehend aus: 70 g N-Vinylphthalimid; 12,3 g Divinylbenzol 63,5%ig; 1,225 g Azobisisobutyronitril und 30 ml Dichlorethan. Man polymerisiert 3 Stunden bei 60°C und 16 Stunden bei 80°C. Nach beendeter Polymerisation wird das entstandene Perlpolymerisat abgenutscht und gründlich mit destilliertem Wasser und Methanol gewaschen. Trockenausbeute: 80,7 g ≙ 98,06 Gew.-%.

Beispiel 7

In einem Reaktionsgefäss, bestückt mit Thermometer, Rührer und Rückflusskühler, werden 77 ml destilliertes Wasser und 0,077 g Methylcellulose vorgelegt und auf 60°C erwärmt. Dazu gibt man unter Rühren die gelöste organische Phase, bestehend aus: 20 g N-Vinylphthalimid; 3 g Divinylbenzol 63,5%ig; 1 g Styrol, 0,35 g Azobisisobutyronitril und 10 ml Dichlorethan. Man polymerisiert 3 Stunden bei 60°C und 16 Stunden bei 80°C. Nach beendeter Polymerisation wird das entstandene Perlpolymerisat über eine Nutsche abfiltriert und gründlich mit destilliertem Wasser und Methanol gewaschen. Trockenausbeute: 21,2 g ≙ 88,33 Gew.-%.

Beispiel 8

In einem Reaktionsgefäss, bestückt mit Thermometer, Rührer und Rückflusskühler werden 77 ml destilliertes Wasser; 0,077 g Methylcellulose und 0,01 g $NaNO_2$ vorgelegt und auf 60°C erwärmt. Dazu gibt man unter Rühren die gelöste organische Phase, bestehend aus: 20 g N-Vinylphthalimid; 3,5 g Divinylbenzol 63,5%ig; 0,35 g Azobisisobutyronitril und 10 ml Dichlorethan. Man polymerisiert 3 Stunden bei 60°C, 12 Stunden bei 80°C und 4 Stunden bei 90°C. Nach beendeter Polymerisation wird das entstandene Perlpolymerisat abgenutscht und gründlich mit destilliertem Wasser und Methanol gewaschen. Trockenausbeute: 94,89 Gew.-%.

Verseifung der Perlpolymerisate

Beispiel 9

30 g des nach Beispiel 6 hergestellten Perlpolymerisates werden mit 100 g Hydrazinhydrat (64 gew.-%ig) versetzt, 2 Stunden lang quellen gelassen und anschliessend 24 Stunden lang auf Rückflusstemperatur erhitzt. Es entstehen 10,2 g hydrazinolysierter Austauscher. Der Austauscher besitzt eine schwach basische Totalkapazität von 11,72 meq/g und eine schwach saure Totalkapazität von 1,64 meq/g.

Beispiel 10

30 g des nach Beispiel 6 hergestellten Perlpolymerisates werden mit 300 g 20%iger Natronlauge versetzt, über Nacht quellen gelassen, dann im Autoklaven in 1 Stunde auf 100°C, in einer weiteren Stunde auf 180°C erhitzt und bei dieser Temperatur 4 Stunden lang gehalten. Es entstehen 22,8 g Austauscher mit einer schwach basischen Totalkapazität von 0,598 Äq/l und einer schwach sauren Totalkapazität von 0,97 Äq/l.

**Patentansprüche**

1. Verfahren zur Herstellung von cyclischen N-Vinylacylaminen der Formel (I)

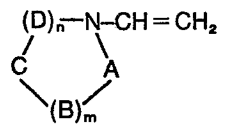

(I),

worin

A und D die CO-Gruppe bedeuten,

B für Sauerstoff oder die NH-Gruppe steht,

C einen gesättigten Kohlenwasserstoffrest mit 1 bis 3 Kohlenstoffatomen oder einen Benzolring, der teilweise oder vollständig hydriert sein kann, darstellt und

m und n unabhängig voneinander 0 oder 1 bedeuten und wobei die Zahl der Ringglieder 5 oder 6 ist,

dadurch gekennzeichnet, dass man Kohlensäureester von cyclischen N-Hydroxyethyl-acylaminen der Formel (II) oder (III)

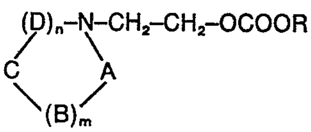

(II)

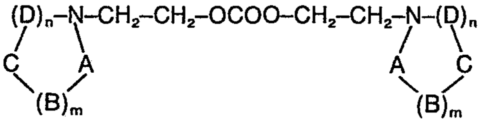
(III),

in denen

A, B, C, D, m und n die zuvor genannte Bedeutung haben und

R für einen gesättigten Kohlenwasserstoffrest mit 1 bis 4 Kohlenstoffatomen steht,

in Gegenwart katalytischer Mengen einer oder mehrerer alkalisch reagierender Verbindungen von Alkali- oder Erdalkalimetallen auf Temperaturen von 120 bis 250°C erhitzt und anschliessend das Reaktionsprodukt durch Destillation abtrennt.

2. Verfahren nach Anspruch 1, dadurch gekennzeichnet, dass man als Kohlensäureester der cyclischen N-2-Hydroxyethyl-acylamine die Carbonate oder die Alkylcarbonate des N-2-Hydroxyethylpyrrolidinons, des N-2-Hydroxyalkyloxazolidinons und des N-2-Hydroxyalkyl-phthalimids einsetzt.

3. Verfahren nach Ansprüchen 1 und 2, dadurch gekennzeichnet, dass man die Reaktion bei Temperaturen von 140 bis 220°C durchführt.

4. Verfahren nach Ansprüchen 1 bis 3, dadurch gekennzeichnet, dass man als alkalisch reagierende Verbindungen die Hydroxide, die Alkoholate, die Carbonate oder die Carboxylate des Natriums oder Kaliums einsetzt.

5. Verfahren nach Ansprüchen 1 bis 4, dadurch gekennzeichnet, dass man die alkalisch reagierenden Verbindungen in Mengen von 0,001 bis 5 Gew.-%, bezogen auf die Menge des Ausgangsmaterials, einsetzt.

6. Verfahren nach Ansprüchen 1 bis 5, dadurch gekennzeichnet, dass man die Reaktionsprodukte durch Destillation bei Drucken von 0,01 bis 200 mbar abtrennt.

## Claims

1. Process for preparing cyclic N-vinylacylamines of the formula (I)

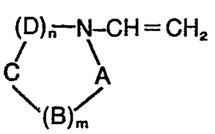
(I),

wherein

A and D denote the CO group,

B represents oxygen or the NH group,

C represents a saturated hydrocarbon radical having 1 to 3 carbon atoms or a benzene ring which can be partially or completely hydrogenated and m and n independently of each other denote 0 or 1 and where the number of ring members is 5 or 6,

characterised in that carbonic acid esters of cyclic N-hydroxyethylacylamines of the formula (II) or (III)

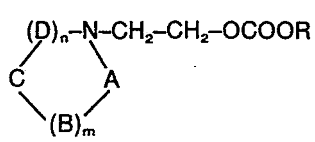
(II)

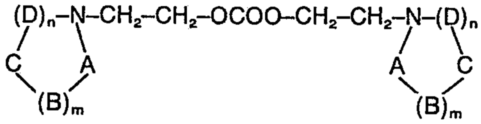
(III),

in which

A, B, C, D, m and n have the previously mentioned meaning and

R represents a saturated hydrocarbon radical having 1 to 4 carbon atoms,

are heated to temperatures of 120 to 250°C in the presence of catalytic amounts of one or more alkali or alkaline earth metal compounds having an alkaline reaction and the reaction product is then separated off by distillation.

2. Process according to Claim 1, characterised in that the carbonates or the alkyl carbonates of N-2-hydroxyethylpyrrolidinone, of N-2-hydroxyalkyloxazolidinone and of N-2-hydroxyalkylphthalimide are used as the carbonic acid esters of cyclic N-2-hydroxyethylacylamines.

3. Process according to Claims 1 and 2, characterised in that the reaction is carried out at temperatures of 140 to 220°C.

4. Process according to Claims 1 to 3, characterised in that the hydroxides, the alcoholates, the carbonates or the carboxylates of sodium or of potassium are used as compounds having an alkaline reaction.

5. Process according to Claims 1 to 4, characterised in that the compounds having an alkaline reaction are used in amounts of 0.001 to 5% by weight, relative to the amount of the starting material.

6. Process according to Claims 1 to 5, characterised in that the reaction products are separated off by distillation under pressures of 0.01 to 200 mbar.

## Revendications

1. Procédé de production de N-vinylacylamines cycliques de formule (I)

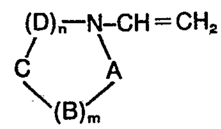
(I),

dans laquelle

A et D représentent le groupe CO,

B représente l'oxygène ou le groupe NH,

C est un reste d'hydrocarbure saturé ayant 1 à 3 atomes de carbone ou un noyau benzénique qui peut être partiellement ou totalement hydrogéné et

m et n ont, indépendamment l'un de l'autre, la valeur 0 ou 1 et le nombre de chaînons du noyau est égal à 5 ou 6,

caractérisé en ce qu'on chauffe à des températures de 120 à 250°C en présence de quantités catalytiques d'un ou plusieurs composés à réaction alcaline de métaux alcalins ou alcalino-terreux des esters d'acides carboniques de N-hydroxyéthylacylamines cycliques de formule (II) ou (III)

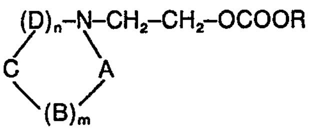

(II)

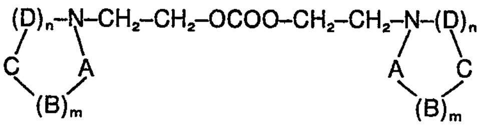

(III),

dans lesquelles

A, B, C, D, m et n ont la définition indiquée précédemment et

R représente un reste d'hydrocarbure saturé ayant 1 à 4 atomes de carbone,

puis on sépare par distillation le produit de réaction.

2. Procédé suivant la revendication 1, caractérisé en ce qu'on utilise comme esters d'acides carboniques des N-2-hydroxyéthylacylamines cycliques les carbonates ou les alkylcarbonates de la N-2-hydroxyéthylpyrrolidinone, de la N-2-hydroxyalkyloxazolidinone et du N-2-hydroxyalkylphtalimide.

3. Procédé suivant les revendications 1 et 2, caractérisé en ce qu'on conduit la réaction à des températures de 140 à 220°C.

4. Procédé suivant les revendications 1 à 3, caractérisé en ce qu'on utilise comme composés à réaction alcaline les hydroxydes, les alcoolates, les carbonates ou les carboxylates du sodium ou du potassium.

5. Procédé suivant les revendications 1 à 4, caractérisé en ce qu'on utilise les composés à réaction alcaline en quantités de 0,001 à 5% en poids par rapport à la quantité de matière de départ.

6. Procédé suivant les revendications 1 à 5, caractérisé en ce que les produits de réaction sont séparés par distillation à des pressions de 0,01 à 200 mbars.